# FASCICULE DE BREVET EUROPEEN

(11) **EP 1 815 844 B1**
(45) Date de publication et mention de la délivrance du brevet: **29.04.2015**
(21) Numéro de dépôt: 06026641.8
(22) Date de dépôt: 22.12.2006
(51) Int. Cl.: A61K 8/81, A61Q 19/00, A61P 17/16, A61K 31/79, A61K 31/78, A61Q 17/00, A61Q 17/04

(54) **Compositions photoprotectrices réduisant les risques de sensibilisation cutanée**
Lichtschutzzusammensetzungen mit vermindertem Risiko der Hautsensibilisierung
Photoprotecting compositions with reduced risk of skin sensitisation

(30) Priorité: 26.12.2005 FR 0513331; 26.12.2005 FR 0513332
(43) Date de publication de la demande: 08.08.2007
(73) Titulaire: Laboratoires Dermatologiques d'Uriage, 92404 Courbevoie Cedex (FR)
(72) Inventeur: Lefeuvre, Luc, 78580 Bazemont (FR); Le Breton, Catherine, 92600 Asnieres sur Seine (FR)
(74) Mandataire: Célanie, Christian

(56) Documents cités:
- EP-A1- 1 051 963
- WO-A1-00/71084
- WO-A1-2004/069216
- DE-A1- 4 243 119
- FR-A- 2 846 555
- FR-A1- 2 799 119
- US-A1- 2004 028 708
- SHIMADA, K. ET AL: "Poly (MPC-co-SMA) for high performance carrier ingredients", SÖFW JOURNAL, CODEN: SOFJEE; ISSN: 0942-7694, vol. 131, no. 9, septembre 2005 (2005-09), pages 16-24, XP009071471,
- PATENT ABSTRACTS OF JAPAN vol. 2003, no. 12, 5 décembre 2003 (2003-12-05) -& JP 2005, 187350, A, (POLA CHEM IND INC), 14 juillet 2005 (2005-07-14)
- OTERI, ROSARIO ET AL: "A new waterproofing agent for sunscreen products", COSMETICS & TOILETRIES , CODEN: CTOIDG; ISSN: 0361-4387, vol. 192, no. 3, 1987, pages 107-109, XP009071492,
- DATABASE CA [Online] CHEMICAL ABSTRACTS SERVICE, COLUMBUS, OHIO, US; 9 juillet 2004 (2004-07-09), INOMATA, KIYOSHI ET AL: "Phosphorylcholine group-containing polymers as film-forming agents for hair preparations", XP002396799, extrait de STN Database accession no. 2004:549697 & JP 2004 189678 A (NOF CORPORATION, JAPAN) 8 juillet 2004 (2004-07-08)
- MERCK KGAA: "Eusolex UV-Pearls OMC - Sunglasses for the Skin", BROCHURE MERCK KGAA, XX, XX, 1 juin 2004 (2004-06-01), page 1, XP002397966,

## Description

La présente invention se rapporte au domaine des nécessités de la vie et en particulier à la protection de la peau.

Elle a plus particulièrement pour objet des compositions photoprotectrices réduisant les risques de sensibilisation cutanée, contenant une association de copolymère de Vinylpyrrolidone (PVP) et de copolymère de 2-méthacryloyloxyéthyl phosphoryl choline (poly MPC) en tant qu'agent renforçant la barrière cutanée.

Elle a spécifiquement pour objet des compositions photoprotectrices réduisant les risques de sensibilisation cutanée, contenant une association de copolymère de Vinylpyrrolidone/Triacontène ou de Vinylpyrrolidone/Eicosène et de copolymère de 2-méthacryloyloxyéthyl phosphorylcholine/méthacrylate d'alkyle ou de copolymère de 2-méthacryloyloxyéthyl phosphoryl choline/chlorure de 2-hydroxy 3-méthacryloyl oxypropyle triméthylammonium, comme agent protecteur renforçant ou reconstituant la barrière cutanée, en association ou en mélange avec un ou plusieurs filtres anti-UV et un ou plusieurs excipients ou véhicules inertes, non toxiques, dermatologiquement acceptables.

Les zones de peau naturellement exposées à la lumière ou qui subissent fréquemment une surexposition à la lumière, nécessitent l'utilisation de produits photoprotecteurs afin d'éviter les effets néfastes du rayonnement solaire, comme par exemple :
- les érythèmes solaires ;
- le photovieillissement induit ;
- le risque d'apparition de cancers cutanés (mélanomes, épithéliomas) ;
- la lucite estivale bénigne (« allergie solaire »).

Cependant même s'il existe à l'heure actuelle des produits apportant une photoprotection efficace, la plupart d'entre eux ont des formulations à base de filtres solaires organiques. Ces derniers possèdent un certain pouvoir sensibilisant, pouvant induire la survenue d'allergies cutanées après usage répété.

On a montré que la sensibilisation nécessite au préalable un passage du composé organique à travers la barrière cutanée, pour entrer en contact avec les cellules vivantes de l'épiderme, notamment avec les cellules de Langherhans appelées « cellules sentinelles ».

Afin d'éviter une telle sensibilisation aux produits organiques utilisés en cosmétique, il a donc semblé particulièrement intéressant d'empêcher le plus possible, de préférence totalement, leur pénétration à travers la barrière cutanée.

D'autre part, la pénétration cutanée de certains produits cosmétiques peut entraîner à long terme des problèmes de toxicité, par un phénomène d'accumulation ( Hayden et. al., Skin Pharmacol. Physiol. 2005, 18(4), 170-174 ; Kunz et. al., Toxicol. Sci. 2006, 90(2), 349-361). Il est donc également intéressant pour cette raison de limiter la pénétration de produits organiques à travers la barrière cutanée.

Le problème technique à résoudre était la mise au point de compositions photoprotectrices renforçant ou reconstituant la barrière cutanée, qui permettent ainsi de limiter, voire même d'empêcher complètement la pénétration de composés organiques allergisants tels que les filtres solaires. Les phénomènes ou processus de sensibilisation cutanée liés à ces composés sont alors évités.

Ce problème a été résolu d'une manière inattendue par l'utilisation de copolymère de Vinylpyrrolidone/Triacontène ou de Vinylpyrrolidone/Eicosène, associé à un copolymère de 2-méthacryloyloxy-éthylphosphorylcholine/ méthacrylate d'alkyle (poly MPC) ou à un copolymère de 2-méthacryloyloxyéthyl phosphorylcholine/chlorure de 2-hydroxy 3-méthacryloyl oxypropyle triméthylammonium, sous forme de compositions photoprotectrices, en mélange ou en combinaison avec un ou plusieurs filtres anti-UV et un ou plusieurs excipients ou véhicules solides, pâteux ou liquides, inertes, non toxiques, dermatologiquement acceptables.

Les polymères de Vinylpyrrolidone ont la propriété de réduire fortement les sensibilisations induites par des substances irritantes, notamment par les phénols, molécules chimiquement proches de nombreux filtres organiques anti-solaires.

Une étude récente, effectuée avec 1e MPC seul, a montré qu'il permettait de limiter la pénétration d'un filtre solaire organique tel que le p-méthoxycinnamate de 2-éthylhexyle (OMC).

L'association PVP - poly MPC a déjà été décrite et revendiquée, notamment dans la demande de brevet français 2 846 555 publiée le 7 mai 2004 au nom de la Demanderesse en tant que barrière épidermique, pour limiter ou supprimer les effets irritants pour la peau de différentes substances. Toutefois, ce document ne décrit pas l'incorporation de cette association dans une composition photoprotectrice.

De plus, la présente invention préconise l'utilisation de copolymères de PVP plutôt que d'homopolymères. Les résultats obtenus en tant que protecteur de la peau indiquent que l'association des copolymères PVP/Triacontène ou PVP/Eicosène et d'un copolymère de poly MPC/méthacrylate d'alkyle conduit à un effet complémentaire entre ces deux produits.

Les compositions selon l'invention jouent un rôle important comme restructurant pour maintenir l'intégrité de la peau et comme protecteur contre les allergies et les irritations cutanées.

En outre, cette association possède des propriétés dermatologiques et/ou cosmétiques favorables :
- facteur de rétention d'eau,
- limitation de la "perspiration" cutanée en eau,
- protection de la peau contre les facteurs irritants (produits détergents, substances acides, etc...),
- intégration dans la barrière cutanée jusqu'à 10 µm de profondeur, pour réparer les dommages.

Dans ce qui précède, le terme alkyle indique un radical hydrocarboné ayant de 1 à 22 atomes de carbone. Ces alkyles peuvent être par exemple : le méthyle, l'éthyle, le butyle, l'isobutyle, le terbutyle, le néopentyle, l'hexyle, le dodécyle, l'hexadécyle, l'octadécyle.

Parmi les copolymères de 2-méthacryloyloxyéthyl phosphorylcholine/méthacrylate d'alkyle, on pourra citer en particulier le copolymère de 2-méthacryloyloxyéthyl phosphorylcholine et de méthacrylate de *n*-butyle, également connu sous la dénomination INCI Polyquaternium-51 ; ou encore le copolymère de 2-méthacryloyloxyéthyl phosphorylcholine et de méthacrylate de stéaryle, également connu sous la dénomination INCI Polyquaternium-61.

Dans le cas des compositions contenant le copolymère de Vinylpyrrolidone/Triacontène ([136445-69-7]), on utilisera de préférence le produit de qualité cosmétique commercialisé sous la marque ANTARON® WP-660.

Dans le cas des compositions contenant le copolymère de Vinylpyrrolidone/Eicosène ([28211-18-9]), on utilisera de préférence le produit de qualité cosmétique commercialisé sous la marque ANTARON® V-220F.

Dans les compositions selon l'invention, la teneur en agent anti-sensibilisation varie de 0,005 % à 10 % en masse par rapport à la masse totale des compositions, à savoir de 0,1 à 10 % de copolymère PVP/Triacontène ou PVP/Eicosène et de 0,005 % à 10 % de copolymère de 2-méthacryloyloxyéthyl phosphorylcholine.

Cette association de polymères permet d'assurer à la peau une barrière protectrice efficace vis-à-vis des produits potentiellement allergisants. Toutefois, pour les compositions s'adressant aux personnes particulièrement sensibles, ou nécessitant l'obtention d'une texture particulière, on pourra choisir d'ajouter un ou plusieurs polymères complémentaires.

Parmi les polymères pouvant être ajoutés aux compositions selon l'invention, on peut citer par exemple le Polyperfluoromethylisopropyl Ether (dénomination INCI). Il est notamment commercialisé sous la marque Fomblin® HC.

On peut également citer le polymère réticulé acide adipique / diéthylène glycol / glycérol (dénomination INCI: Crosspolymer Adipic Acid/Diethylene Glycol/Glycerin). Il est notamment commercialisé sous la marque Lexorez® 100.

On peut encore citer le Polysilicone-8 (dénomination INCI). Il est notamment commercialisé sous la marque Silicones Plus Polymer VS80 Dry.

Ces exemples illustrent les polymères pouvant être introduits à titre complémentaire dans les compositions selon l'invention. Ils ne sont nullement limitatifs.

On pourra introduire un ou plusieurs polymères complémentaires en quantités variant par exemple de 0,005 % à 10 % en masse par rapport à la masse totale des compositions.

Selon l'invention, l'association des copolymères PVP/Triacontène ou PVP/Eicosène et poly MPC/méthacrylate d'alkyle ou poly MPC/chlorure de 2-hydroxy 3-méthacryloyl oxypropyle triméthylammonium est incorporée à des compositions photoprotectrices, notamment destinées à la protection solaire. Ces dernières contiennent un ou plusieurs filtres anti-UV, notamment des filtres solaires organiques.

Parmi les filtres solaires couramment utilisés en cosmétologie ou en dermatologie, on citera plus particulièrement les esters d'acide cinnamique, comme par exemple le *p-*méthoxycinnamate d'isoamyle ou encore le *p*-méthoxycinnamate de 2-éthylhexyle (INCI: Ethyl Hexyl Methoxycinnamate), notamment commercialisé sous le nom d'Eusolex® 2292 ; les dérivés du dibenzoyl méthane, comme le Butyl Methoxydibenzoyl Methane (Eusolex® 9020) ; les dérivés de la 1,3,5-triazine-2,4,6-triamine, comme par exemple l'Ethyl Hexyl Triazone (UVINUL® T 150) ; le Diethylamino Hydroxybenzoyl Hexyl Benzoate (UVINUL® A+); la Bis-ethyl Hexyloxyphenol Methoxyphenol Triazine (Tinosorb® S); l'Octocrylene (Eusolex® 1020) ; le Dimethicodiethylbenzalmalonate (Parsol® SLX); la Di-ethylhexyl-butamido Triazone (Uvasorb® HEB) ; les dérivés du phényl benzimidazole, comme par exemple le Phenylbenzimidazole Sulfonic Acid, (Eusolex® 232) ; les dérivés de la benzophénone, comme la Benzophenone-3 (Tinosorb® B3), la Benzophenone-4 (UVINUL® MS 40) ou la Benzophenone-5 ; les dérivés de l'acide *p*-aminobenzoïque, comme le dérivé éthoxylé PEG 25 - PABA (UVINUL® P25); le Methylene Bis-benzotriazolyl Tetramethylbutylphenol (Tinosorb® M).

Les composés ci-dessus sont cités sous leur dénomination INCI.

Les compositions selon l'invention sont particulièrement intéressantes pour limiter la pénétration de filtres solaires dont la taille et la structure leur permettraient de traverser facilement l'épiderme. On pourra citer par exemple l'Octocrylene, l'Ethyl Hexyl Triazone, l'Ethyl Hexyl Methoxycinnamate et le Butyl Methoxydibenzoyl Methane.

Selon une forme préférentielle de l'invention, les compositions pour la protection solaire contiennent au moins les filtres anti-UV suivants : Octocrylene, Ethyl Hexyl Triazone, Methylene Bis-benzotriazolyl Tetramethylbutylphenol et Butyl Methoxy-dibenzoyl Methane. Cette association permet d'obtenir une couverture optimale du spectre UV.

Selon une autre forme préférentielle de l'invention, les compositions pour la protection solaire contiennent au moins les filtres anti-UV suivants : Ethyl Hexyl Methoxycinnamate, Ethyl Hexyl Triazone, Diethylamino Hydroxybenzoyl Hexyl Benzoate, Bis-ethyl Hexyloxyphenol Methoxyphenol Triazine, Methylene Bis-benzotriazolyl Tetramethylbutylphenol. Cette association permet également d'obtenir une couverture optimale du spectre UV.

Il est connu que la présence simultanée de plusieurs filtres solaires dans des compositions photoprotectrices peut conduire à une réaction chimique de dégradation de ces filtres. Le brevet européen EP 0 815 835 et la demande française FR 05 13332 mettent notamment en évidence la dégradation photo-induite du Butyl Methoxy-dibenzoyl Methane en présence d'Ethyl Hexyl Methoxycinnamate.

En conséquence, selon une autre forme préférentielle de l'invention, les compositions pour la protection solaire contiennent au moins un filtre anti-UV sous forme micro-encapsulée, ce filtre anti-UV étant choisi parmi le Butyl Methoxy-dibenzoyl Methane et l'Ethyl Hexyl Methoxycinnamate.

De manière davantage préférée, ces microcapsules sont formées d'oxydes inorganiques, comme par exemple d'un gel de silice. On peut notamment utiliser l'Ethyl Hexyl Methoxycinnamate micro-encapsulé commercialisé sous la dénomination Eusolex® UV-Pearls™ OMC.

Cette micro-encapsulation assure la stabilité des filtres anti-UV encapsulés, en empêchant les réactions chimiques avec les filtres se trouvant sous forme libre. Elle a également l'avantage de réduire la pénétration cutanée de ces filtres encapsulés.

Selon une autre forme préférentielle de l'invention, les compositions pour la protection solaire contiennent au moins les filtres anti-UV suivants : Octocrylene, Ethyl Hexyl Triazone, Ethyl Hexyl Methoxycinnamate et Butyl Methoxydibenzoyl Methane, le Butyl Methoxy-dibenzoyl Methane et/ou l'Ethyl Hexyl Methoxycinnamate se trouvant sous forme micro-encapsulée. Cette association permet également d'obtenir une couverture optimale du spectre UV.

Les compositions selon l'invention contiennent un ou plusieurs filtres solaires, chacun en quantité allant préférentiellement de 0,1 à 10 % en masse et, de manière davantage préférée, de 1 à 5 % en masse, par rapport à la masse totale de la composition.

Les compositions dermatologiques et/ou cosmétologiques selon l'invention peuvent, en outre, renfermer des agents émollients, adoucissants, des agents conservateurs et/ou des parfums.

Les compositions dermatologiques et/ou cosmétologiques selon l'invention se présentent sous une forme appropriée à l'application topique, comme par exemple les gels, les lotions, les émulsions huile dans l'eau ou eau dans l'huile, les dispersions, les laits, les crèmes, les onguents, les mousses, les bâtons (sticks), les sprays et/ou les aérosols.

Ces préparations peuvent être parfumées. On veillera, à cet effet, à ce que le parfum utilisé ne soit pas susceptible d'entraîner des phénomènes de sensibilisation.

Les compositions selon l'invention sont destinées à être appliquées sur la peau, de préférence à raison de 1 à 6 applications par jour. Elles seront préférentiellement appliquées sur le visage et les parties du corps exposés à la lumière ou au soleil.

L'invention a également pour objet les compositions photoprotectrices précédemment décrites, pour leur utilisation pour fournir une protection solaire efficace sans induire de sensibilisation cutanée aux filtres anti-UV.

Les exemples suivants illustrent l'invention.

### EXEMPLE I

### Crème barrière photoprotectrice huile dans eau

| **Ingrédients (Nomenclature INCI)** | **% massique** |
|---|---|
| C20-22 Alkyl phosphate / C20-22 alcohols | 0,5-6 |
| Diethylamino Hydroxybenzoyl Hexyl Benzoate | 5 |
| Dicaprylyl Carbonate | 5-20 |
| Bis-ethyl Hexyloxyphenol Methoxyphenol Triazine | 3 |
| Ethyl Hexyl Triazone | 5 |
| Ethyl Hexyl Methoxycinnamate | 7,5 |
| Tricontanyl/PVP | 2 |
| Eau thermale d'Uriage | 10 |
| Tetrasodium EDTA | 0,2 |
| Xanthan gum | 0,01-0,4 |
| Benzoic acid | 0,2 |
| Glycerin | 2 |
| Polyquaternium-51 | 0,005-5 |
| Sodium Hydroxide | 0,017 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Butylene glycol | 1 |
| Chlorphenesin | 0,3 |
| o-Cymen-5-ol | 0,1 |
| Cyclomethicone | 5 |
| Parfum | 0,35 |
| Eau purifiée | q.s.p.100 |

### EXEMPLE II

### Crème barrière photoprotectrice eau dans huile

| **Ingrédients (Nomenclature INCI)** | **% massique** |
|---|---|
| PEG-30 Dipolyhydroxystearate | 1-5 |
| Polyglyceryl-3 Diisostearate | 0,5-3 |
| Dicaprylyl Carbonate | 5-20 |
| Beeswax | 0,2 |
| Polyquaternium-61 | 0,01-0,5 |
| Diethylamino Hydroxybenzoyl Hexyl Benzoate | 5 |
| Bis-ethyl Hexyloxyphenol Methoxyphenol Triazine | 3 |
| Ethyl Hexyl Triazone | 5 |
| Ethyl Hexyl Methoxycinnamate | 7,5 |
| Tricontanyl/PVP | 2 |
| Acrylates / C10-C30 Alkyl Acrylates Crosspolymer | 0,01-1 |
| Eau thermale d'Uriage | 10 |
| Tetrasodium EDTA | 0,2 |
| Benzoic acid | 0,2 |
| Sodium Chloride | 1 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Butylene glycol | 1 |
| Chlorphenesin | 0,3 |
| o-Cymen-5-ol | 0,1 |
| Eau purifiée | q.s.p. 100 |

### EXEMPLE III

### Crème barrière photoprotectrice huile dans eau

| **Ingrédients (Nomenclature INCI)** | **% massique** |
|---|---|
| C20-22 Alkyl phosphate / C20-22 alcohols | 0,5-6 |
| Butyl Methoxydibenzoyl Methane | 5 |
| Dicaprylyl Carbonate | 5-20 |
| Octocrylene | 5 |
| Ethyl Hexyl Triazone | 5 |
| Tricontany1/PVP | 2 |
| Eau thermale d'Uriage | 10 |
| Tetrasodium EDTA | 0,2 |
| Xanthan gum | 0,01-0,4 |
| Benzoic acid | 0,2 |
| Glycerin | 2 |
| Polyquaternium-51 | 0,005-5 |
| Sodium Hydroxide | 0,02 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Butylene glycol | 1 |
| Chlorphenesin | 0,3 |
| *o*-Cymen-5-ol | 0,1 |
| Cyclomethicone | 4 |
| Parfum | 0,35 |
| Eau purifiée | q.s.p. 100 |

### EXEMPLE IV

### Crème barrière photoprotectrice huile dans eau

### Cette crème correspond à la dénomination BARIESUN H/E de l'exemple VI

| **Ingrédients (Nomenclature INCI)** | **% massique** |
|---|---|
| C20-22 Alkyl phosphate / C20-22 alcohols | 1,5 |
| Dicaprylyl Carbonate | 18 |
| Tricontanyl/PVP | 2 |
| Polydecene | 1 |
| Butyl Methoxydibenzoyl Methane | 5 |
| Octocrylene | 5 |
| Ethyl Hexyl Triazone | 5 |
| Eau thermale d'Uriage | 10 |
| Ascorbyl Tetraisopalmitate | 0,01 |
| Polyperfluoroethoxymethoxydifluoromethyl distearamide | 2 |
| Xanthan gum | 0,08 |
| Tetrasodium EDTA | 0,2 |
| Glycerin | 2 |
| Glucose | 0,45 |
| Tréhalose | 0,05 |
| Benzoic acid | 0,2 |
| Polyquaternium-51 | 4 |
| Sodium Hydroxide | 0,02 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Butylene glycol | 1 |
| Chlorphenesin | 0,3 |
| *o*-Cymen-5-ol | 0,1 |
| Parfum | 0,35 |
| Eau purifiée | q.s.p. 100 |

### EXEMPLE V

### Crème barrière photoprotectrice eau dans huile

### Cette crème correspond à la dénomination BARIESUN E/H de l'exemple VI

| **Ingrédients (Nomenclature INCI)** | **% massique** |
|---|---|
| PEG-30 Dipolyhydroxystearate | 4 |
| Polyglyceryl-3 Diisostearate | 2 |
| Dicaprylyl Carbonate | 19 |
| Beeswax | 0,3 |
| Tricontanyl/PVP | 2 |
| Butyl Methoxydibenzoyl Methane | 5 |
| Octocrylene | 5 |
| Ethyl Hexyl Triazone | 5 |
| Diethylamino Hydroxybenzoyl Hexyl Benzoate | 5 |
| Tocopheryl acetate | 0,1 |
| Xanthan gum | 0,2 |
| Eau thermale d'Uriage | 10 |
| Tetrasodium EDTA | 0,2 |
| Benzoic acid | 0,2 |
| Sodium Chloride | 1 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Polyperfluoromethylisopropyl ether | 1 |
| Polyquaternium-51 | 4 |
| Chlorphenesin | 0,3 |
| *o*-Cymen-5-ol | 0,1 |
| Eau purifiée | q.s.p. 100 |

### EXEMPLE VI

### Etude de la diffusion in vitro à travers la peau humaine de plusieurs filtres solaires, dans différentes formulations cosmétiques

### BUT DE L'ETUDE

Cette étude a pour but d'étudier la diffusion d'un ou plusieurs filtres solaires, incorporé dans trois formulations différentes à la même concentration finale, à travers la peau humaine.

### MODE OPERATOIRE

### Résumé de l'étude

L'expérience a été réalisée sur une peau humaine issue d'une plastie abdominale de femme de type caucasien, âgée de 52 ans. Cette plastie a, préalablement, été congelée à -20°C.

Les explants de peau ont été placés sur une cellule de Franz en diffusion passive et les formulations ont été appliquées de façon non occlusive.

Pour chaque cellule, 250 µl de crème ont été déposés sur la peau, soit 142 mg/cm².
Le fluide receveur était du PBS (phospahte buffered salin solution) additionné de 1% BSA (bovin serum albumin), maintenu à 32.5°C par un circuit thermostaté.
3 formulations ont été testées et 3 cellules ont servies de témoin.
Il y a 2 séries d'application qui ont été identifiées M1 et M2.
Le temps de contact total a été de 24h.
Pour chaque cellule, un échantillon (1.5ml) du fluide receveur a été prélevé aux temps 0h, 8h et 24h.
Pour chaque cellule, le restant du produit appliqué a été prélevé et la surface de la peau a été rincée.
Pour chaque cellule, six strippings ont été réalisés.
Pour chaque cellule, le restant de stratum comeum, épiderme et derme ont été prélevés.

Après extraction, les filtres solaires ont été dosés dans chaque prélèvement, par des méthodes HPLC.

### Mode opératoire

### 1. Préparation des produits

3 produits contenant les mêmes concentrations de 3 filtres solaires.
- Filtres solaires :: Octocrylene (OCR) à 5% = Eusolex® OCR Butylmethoxydibenzoylmethane (BMDBM) à 5% = Eusolex® 9020 Ethylhexyltriazone (OCT) à 5% = Uvinul® T 150

### Produit P1 BASE H/E

| **Ingrédients (Nomenclature INCI)** | **% massique** |
|---|---|
| Glyceryl Stearate & PEG -100 Stearate | 2 |
| Dicaprylyl Carbonate | 18 |
| Butyl Methoxydibenzoyl Methane | 5 |
| Octocrylene | 5 |
| Ethyl Hexyl Triazone | 5 |
| Eau thermale d'Uriage | 10 |
| Xanthan gum | 0,6 |
| Tetrasodium EDTA | 0,2 |
| Glycerin | 2 |
| Benzoic acid | 0,2 |
| Potassium Cetyl Phosphate | 1 |
| Methylene Bis-benzotriazolyl Tetramethylbutylphenol/ Aqua/Decyl glucoside/Propylene glycol/ Xanthan gum | 12 |
| Citric acid (monohydrate) | 0,024 |
| Butylene glycol | 1 |
| Chlorphenesin | 0,3 |
| *o*-Cymen-5-ol | 0,1 |
| Eau purifiée | q.s.p. 100 |

### Produit P2: BARIESUN H/E (Exemple IV)

### Produit P3: BARIESUN E/H (Exemple V)

Solvant : Isopropanol , Méthanol
Solution à tester : 3 produits P1, P2 et P3
Conservation : température ambiante

### 2. Préparation de la plastie

Conservée à -20°, décongélation de 6 disques à 4°C, pendant une nuit dans une chambre humide (PBS stérile).

### 3. Préparation de l'appareillage

Vérification des connexions des tubulures.
Allumage du bain-marie à 32,5°C.
Fluide récepteur : PBS + 1% serum albumine

Dépôt d'un aliquot de 10 ml du fluide récepteur dans un tube en verre de 15 ml.
Conservation du tube à -20°C jusqu'au dosage

### 4. Application des produits

Suite à la mise en place de la peau sur les cellules, la peau a été laissée se stabiliser pendant deux heures durant lesquelles, le fluide receveur est régulièrement remplacé.

### Dépôt du produit sur la peau:

Pour prélever et appliquer les produits de manière la plus reproductible, une multipette Eppendorf (pipette à déplacement positif de type seringue) a été utilisée.

Quantité déposée : Pour chaque cellule, 250 µl de crème ont été déposés, soit 142 mg/cm².

Le premier jour (Manip M1) application du produit P1 sur 3 cellules de Franz et du produit P2 sur les 3 autres. Le 2° jour (Manip M2) application du produit P3 sur 3 cellules et les 3 autres cellules ne reçoivent pas de produit.

### 5. Lancement de la manipulation

Lancement du programme n°3, dès la fin de l'application des produits.
Prélèvement, à chaque temps donné et pour chaque cellule de Franz, du liquide receveur dans des flacons en verre d'analyse, 2 ml.

Les flacons sont conservées à -20°C jusqu'au dosage des filtres solaires.

### 6. Prélèvement du reste de produit sur la peau

Après les 24 heures d'exposition, l'excès de produit situé à la surface de la peau est prélevé à l'aide d'une spatule, puis déposé dans un flacon en verre de 20 ml.
La peau (et la spatule) est rincée deux fois avec 500 µl d'isopropanol qui sont ré-aspirés ensuite et déposés dans le flacon
Essuyage de la surface cutanée avec un de coton tige, placé ensuite dans le flacon.
Evaporation de l'isopropanol contenu dans le flacon.
Conservation des flacons à -20°C jusqu'à l'extraction des filtres solaires.

### 7. Prélèvement de la peau et réalisation des strippings

Cette étape fait suite à l'élimination et au rinçage de la surface de la peau.
Les disques de peau sont enlevés des cellules de Franz et la zone d'application du produit est prélevée au scalpel.

### Réalisation des strippings:

Les strippings sont réalisés à l'aide de languettes de prélèvement DIAGNOSKIN®, sur lesquelles 3 prélèvements peuvent êtres réalisés.
Deux séries de 3 strippings sont réalisées, soit deux languettes de prélèvement DIAGNOSKIN®.
Le disque de peau est posé sur la plaque de liège et la languette appliquée pendant 5 secondes. Dépôt de chaque languette (stripping vers le bas et la lumière du tube) dans un tube de 15 ml en verre.

2 strippings vierges sans contact avec la peau ont été réalisés. Les tubes sont conservés à 4°C jusqu'à l'extraction des filtres solaires.

Dépôt du disque de peau après les strippings dans un microtube.
Les tubes sont conservés à -20°C jusqu'à l'extraction des filtres.

### 8. Extraction des filtres dans les différents échantillons

### Extraction des filtres de la peau

Reprise des microtubes contenant la peau (épiderme/derme).
Découpe de la peau au scalpel en tout petits morceaux, dans des boîtes de Pétri. Dépôt des morceaux dans des tubes de verre de 15 ml.

Rinçages de la boîte par 2 x 2 ml d'isopropanol (solvant d'extraction), qui sont versés ensuite dans le tube.

Après sonication, agitation et pressage, l'isopropanol est filtré.
Dans une fiole en verre de dosage, 800µl provenant du filtrat de la peau sont déposés. Les fioles sont conservées à -20° en attente du dosage.

### Extraction des filtres des strippings

Reprise des tubes contenant les strippings
Dépôt de 5 ml de méthanol
Attente 1h à température ambiante
Après sonication et agitation, le contenu du tube est filtré (0.45µm) et le tube rincé avec 2 x 1 ml de méthanol qui sont également filtrés.
La seringue est rincée avec 2 x 1ml de méthanol qui sont récupérés et filtrés.
Evaporation du méthanol et ajout de 1ml d'isopropanol.
Après agitation, dépôt du volume à doser dans des fioles en verre.

### Extraction des filtres dans le produit restant sur la surface de la peau

Dépôt de 15 ml d'isopropanol
Après sonication et filtration sur papier filtre, le filtrat est dilué au 1/10^{ème}
(1 ml de filtrat dans 9 ml d'isopropanol).

1 ml de cette dilution est conservé à 4° en attente du dosage.

### 9. Dosages

Le dosage des filtres solaires dans les différents prélèvements a été réalisé suivant les protocoles définis lors de l'étude préliminaire et décrit ci-dessous.

### Matériels et méthode

### Matériels

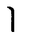 Chaîne HPLC Varian avec détecteur UV

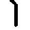 Colonne Xterra longueur 250mm, diamètre interne 4,6 mm, granulométrie 5 µm

### Méthode

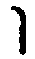 Phase mobile :
- Méthanol contenant 0,1 % de H₃PO₄: 95 volumes
- Eau : 5 volumes

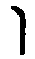 Débit: 1,0 ml/min

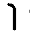 Volume d'injection 10 µl

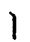 Détection: à 313 nm

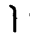 Temps d'analyse 35 minutes

| | OCR | BMDBM | OCT |
|---|---|---|---|
| Limie de détection LD | 0,17 µg/ml | 0,16 µg/ml | 0,58 µg/ml |

### RESULTATS

Les 3 filtres ont été dosés dans les compartiments suivants:
S1 Stratum corneum: stripping 1 à 3.
S2 Stratum corneum: stripping 4 à 6.
ED Le reste du stratum comeum, l'épiderme et le derme.
FR Le milieu receveur aux différents temps de prélèvements (0h, 8h et 24h)
R Le produit restant sur la peau après l'exposition de 24 heures

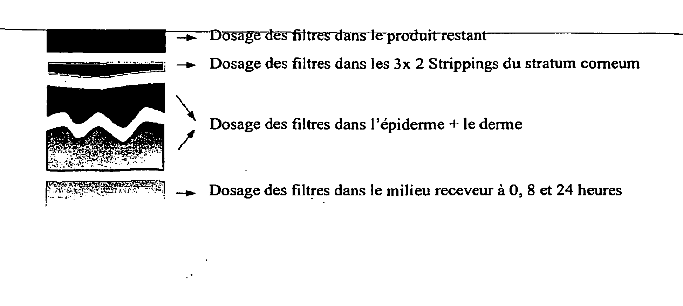

Les compartiments sont représentés graphiquement à la figure 1.
1. Répartition des filtres solaires dans les compartiments investigués, exprimé en % du produit dosé (produit Restant + Startum Corneum + Epiderme + Derme + Fluide Receveur)

**Tableau 1 : Résultats pour l'OCR**

| | **Base H/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **0,04** | 0,00 | **0,05** | 0,02 | **0,06** | 0,01 |
| Stripping 4 à 6 | **0,22** | 0,02 | **0,18** | 0,06 | **0,40** | 0,00 |
| Epiderme+derme | **0,49** | 0,13 | **0,42** | 0,11 | **0,47** | 0,27 |
| Fluide receveur | **0,00** | 0,00 | **0,00** | 0,00 | **0,00** | 0,00 |
| Produit Restant | **99,3** | 0,4 | **99,3** | 1,3 | **99,1** | 1,5 |

**Tableau 2 : Résultats pour le BMDBM**

| | **Base H/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **0,05** | 0,00 | **0,06** | 0,02 | **0,06** | 0,01 |
| Stripping 4 à 6 | **0,24** | 0,01 | **0,19** | 0,06 | **0,34** | 0,05 |
| Epiderme+derme | **0,61** | 0,29 | **0,48** | 0,13 | **0,65** | 0,27 |
| Fluide receveur | **0,00** | 0,00 | **0,00** | 0,00 | **0,00** | 0,00 |
| Produit Restant | **99,1** | 0,6 | **99,3** | 0,9 | **98,9** | 1,6 |

**Tableau 3 : Résultats pour l'OCT**

| | **Base H/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **0,04** | 0,01 | **0,05** | 0,02 | **0,05** | 0,00 |
| Stripping 4 à 6 | **0,16** | 0,01 | **0,14** | 0,05 | **0,31** | 0,08 |
| Epiderme+derme | **0,43** | 0,19 | **0,46** | 0,05 | **0,62** | 0,33 |
| Fluide receveur | 0,00 | 0,00 | 0,00 | 0,00 | 0,00 | 0,00 |
| Produit Restant | 99,4 | 0,7 | 99,4 | 0,4 | 99,0 | 1,0 |

**Tableau 4 : Réduction de la pénétration des filtres solairesdans le compartiment Epiderme + Derme pour le Bariesun H/E par rapport à la Base H/E (en % par rapport aux valeurs de la Base H/E)**

| | OCR | BMDBM | OCT |
|---|---|---|---|
| Réduction de pénétration | 14% | 21% | 7% |

On constate que la formulation Bariesun H/E selon l'invention permet une diminution significative de la pénétration des filtres solaires dans le compartiment Epiderme + Derme par rapport à la base H/E ne contenant pas de complexe de polymères.

### 2. Répartition des filtres dans les compartiments investigués, exprimé en % du produit ayant pénétré

**Tableau 4 : Résultats pour l'OCR**

| | **Base H/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **5,91** | 0,50 | **8,34** | 2,65 | **6,69** | 1,41 |
| Stripping 4 à 6 | **28,77** | 2,30 | **27,34** | 8,72 | **43,19** | 0,00 |
| Epiderme+derme | **65,32** | 17,36 | **64,32** | 17,28 | **50,11** | 29,01 |
| Fluide receveur | **0,0** | 0,0 | **0,0** | 0,0 | **0,0** | 0,0 |

**Tableau 5 : Résultats pour le BMDBM**

| | **BaseH/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **5,07** | 0,37 | **7,71** | 2,35 | **5,84** | 1,24 |
| Stripping 4 à 6 | **27,02** | 1,06 | **26,66** | 8,40 | **32,01** | 4,83 |
| Epiderme+derme | **67,90** | 31,96 | **65,63** | 17,99 | **62,15** | 25,93 |
| Fluide receveur | **0,0** | 0,0 | **0,0** | 0,0 | **0,0** | 0,0 |

**Tableau 6 : Résultats pour l'OCT**

| | **Base H/E** | | **Bariesun H/E** | | **Bariesun E/H** | |
|---|---|---|---|---|---|---|
| | **Moy** | E t | **Moy** | E t | **Moy** | E t |
| Stripping 1 à 3 | **6,48** | 0,82 | **7,65** | 2,37 | **5,21** | 0,25 |
| Stripping 4 à 6 | **24,88** | 1,80 | **21,58** | 7,79 | **31,17** | 8,20 |
| Epiderme+derme | **68,64** | 30,04 | **70,77** | 8,38 | **63,62** | 34,09 |
| Fluide receveur | **0,0** | 0,0 | **0,0** | 0,0 | **0,0** | 0,0 |

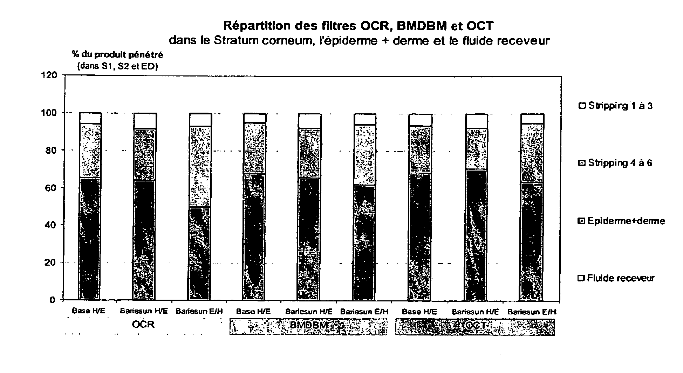

Les résultats sont représentés graphiquement à la figure 2.

On constate que, d'une manière générale, la répartition des filtres solaires est plus importante dans les couches cornées (Stripping 1 à 3 et 4 à 6) pour les compositions Bariesun que pour la base H/E. Les compositions selon l'invention sont donc favorables au maintien des filtres dans les zones cutanées moins susceptibles de réagir aux composés allergisants.

## Revendications

1. Compositions photoprotectrices, **caractérisées en ce qu'**elles contiennent, comme agent anti-sensibilisation, un copolymer de Vinylpyrrolidone/Triacontène ou de Vinylpyrrolidone/Eicosène et un copolymère de 2-méthacryloyloxyéthyl phosphorylcholine/méthacrylate d'alkyle ou de 2-méthacryloyloxyéthyl phosphorylcholine/chlorure de 2-hydroxy 3-méthacryloyl oxypropyle triméthylammonium, en association ou en mélange avec un ou plusieurs filtres anti-UV organiques et un ou plusieurs excipients ou véhicules, inertes, non toxiques, dermatologiquement acceptables.

2. Compositions photoprotectrices selon la revendication 1, dans lesquelles le copolymère de 2-méthacryloyl oxyethylphosphoryl choline / méthacrylate d'alkyle est le composé correspondant à la dénomination INCI Polyquatemium - 61.

3. Compositions photoprotectrices selon la revendication 1 ou la revendication 2, contenant de 0,1 à 10 % de copolymère de Vinylpyrrolidone/Triacontène ou de copolymère de Vinylpyrrolidone/Eicosène.

4. Compositions photoprotectrices selon l'une des revendications 1 à 3, dans lesquelles la quantité de copolymère de méthacryloyloxyéthyl phosphorylcholine s'échelonne de 0,005 % à 10%.

5. Compositions photoprotectrices selon l'une des revendications 1 à 4, contenant en outre un ou plusieurs polymères complémentaires.

6. Compositions photoprotectrices selon la revendication 5, dans lesquelles le ou les polymères complémentaires sont choisis parmi le Polyperfluoromethylisopropyl Ether, le Crosspolymer Adipic Acid/Diethylene Glycol/Glycerin et le Polysilicone-8.

7. Compositions photoprotectrices selon l'une des revendications 1 à 6, contenant un ou plusieurs filtres anti-UV choisis dans le groupe formé de :
- les esters d'acide cinnamique;
- les dérivés du dibenzoyl méthane ;
- les dérivés de la 1,3,5-triazine-2,4,6-triamine ;
- le Diethylamino Hydroxybonzoyl Hexyl Benzoate;
- la Bis-ethyl Hexyloxyphenol Methoxyphenol Triazine ;
- l'Octocrylene ;
- le Dimethicodiethylbenzalmalonate ;
- la Di-ethylhexyl-butamido Triazone ;
- les dérivés de la benzophénone ;
- les dérivés du phényl benzimidazole ;
- les dérivés de l'acide p-aminobenzoïque ;
- le Methylene-bis-benzotriazolyl Tetramethylbutylphenol

8. Compositions photoprotectrices selon la revendication 7, contenant au moins un filtre anti-UV sous forme microencapsulée, ce filtre anti-UV étant choisi parmi le Butyl Methoxydibenzoyl Methane et l'Ethyl Hexyl Methoxycinnamate.

9. Compositions photoprotectrices selon l'une des revendications 1 à 8, sous forme de gel, de lotion, d'émulsion huile dans eau ou eau dans l'huile, de dispersion, de lait, de crème, d'onguent, de mousse, de bâton (sticks), de spray, d'aérosols ou sous toute autre forme appropriée à l'application topique.

10. Composition cosmétique selon l'une des revendications 1 à 9, pour son utilisation pour fournir une protection solaire efficace sans induire de sensibilisation cutanée aux filtres anti-UV.

## Patentansprüche

1. Lichtschutz-Zusammensetzungen, **dadurch gekennzeichnet, dass** sie als Anti-Sensibilisierungsmittel ein Kopolymer von Vinylpyrrolidon/Triacontanol oder von Vinylpyrrolidon/Eicosen und ein Kopolymer von 2-Methacryloyloxyethyl Phosphorylcholin/ Alkylmethacrylat oder von 2-Methacryloyloxyethyl Phosphorylcholin von 2-Hydroxy 3-Methacryloyl Oxypropyl Trimethylammoniumchlorid in Verknüpfung oder im Gemenge mit einem oder mehreren organischen UV-Schutzfiltern und einem oder mehreren inaktiven, nichttoxischen, dermatologisch akzeptablen Grundmassen oder Stoffträgern enthalten.

2. Lichtschutz-Zusammensetzungen nach Anspruch 1, bei denen das Kopolymer von 2-Methacryloyl Oxyethylphosphorylcholin / Alkylmethacrylat eine Zusammensetzung ist, welche der Bezeichnung nach INCI Polyquaternium-61 entspricht.

3. Lichtschutz-Zusammensetzungen nach Anspruch 1 oder Anspruch 2, enthaltend 0,1 bis 10% des Kopolymers von Vinylpyrrolidon/Triacontanol oder des Kopolymers von Vinylpyrrolidon/Eicosen.

4. Lichtschutz-Zusammensetzungen nach einem der Ansprüche 1 bis 3, bei welchen sich die Menge an Kopolymer von Methacryloyloxyethyl Phosphorylcholin von 0,005% bis 10% staffelt.

5. Lichtschutz-Zusammensetzungen nach einem der Ansprüche 1 bis 4, außerdem enthaltend ein oder mehrere komplementäre Polymere.

6. Lichtschutz-Zusammensetzungen nach Anspruch 5, bei denen das oder die komplementären Polymere aus dem Polyperfluoromethyl-Isopropylether, dem Adipic Acid/Diethylene Glycol/Glycerin Crosspolymer und dem Polysilikon-8 ausgewählt werden.

7. Lichtschutz-Zusammensetzungen nach einem der Ansprüche 1 bis 6, enthaltend ein oder mehrere UV-Schutzfilter, welche aus der Gruppe ausgewählt werden, welche gebildet wird aus:
- den Zimtsäureestern;
- den Derivaten von Dibenzoylmethan;
- den Derivaten von 1,3,5-Triazin-2,4,6-Triamin;
- dem Diethylamino-Hydroxybenzoyl-Hexyl-Benzoat;
- dem Bis-Ethylhexyloxyphenol Methoxyphenol Triazin;
- dem Octocrylen;
- dem Dimethicodiethylbenzalmalonat;
- dem Di-Ethylhexyl-Butamido Triazon;
- den Derivaten von Benzophenon;
- den Derivaten von Phenylbenzimidazol;
- den Derivaten *p*-Aminobenzoesäure;
- dem Methylen-Bis-Benzotriazolyl-TetramethylButylphenol.

8. Lichtschutz-Zusammensetzungen nach Anspruch 7, enthaltend mindestens einen UV-Schutzfilter in mikroverkapselter Form, wobei dieser Filter aus Butyl-Methoxydibenzoyl-Methan und Ethylhexyl-Methoxycinnamat ausgewählt wird.

9. Lichtschutz-Zusammensetzungen nach einem der Ansprüche 1 bis 8 in Form eines Gels, einer Lotion, einer Emulsion von Öl in Wasser oder von Wasser in Öl, einer Dispersion, einer Milch, einer Creme, einer Salbe, eines Schaums, eines Stifts (Sticks), eines Sprays, von Aerosolen oder in ganz anderer Form, welche für topische Anwendung geeignet ist.

10. Kosmetische Zusammensetzung nach einem der Ansprüche 1 bis 9 für deren Verwendung, um einen effizienten Sonnenschutz bereitzustellen, ohne eine Empfindlichkeit der Haut auf die UV-Schutzfilter hervorzurufen.

## Claims

1. Photoprotective formulations, **characterized in that** they contain, by way of an anti-sensitization agent, a copolymer of vinylpyrrolidone/triacontane or vinylpyrrolidone /eicosene copolymer and a copolymer of 2-methacryloyloxyethyl phosphorylcholine/alkyl methacrylate or of 2-methacryloyloxyethyl phosphorylcholinechloride of 2-hydroxy 3-methacryloyl oxypropyl trimethylammonium chloride, in combination or in admixture with one or more organic anti-UV filters, and one or more, inert, non-toxic, dermatologically acceptable excipients or carriers.

2. Photoprotective formulations according to Claim 1, **characterized in that** the copolymer of 2-methacryloyloxyethylphosphoryl choline / alkyl methacrylate is a compound designated by the INCI name Polyquaternium - 61.

3. Photoprotective formulations according to one of Claims 1 or 2, containing from 0.1 to 10% of copolymer of vinylpyrrolidone / triacontane or vinylpyrrolidone / eicosene copolymer.

4. Photoprotective formulations according to one of Claims 1 to 3, **characterized in that** the quantity of copolymer of 2-methacryloyloxyethyl phosphorylcholine ranges from 0.005% to 10%.

5. Photoprotective formulations according to one of Claims 1 to 4, containing one or several additional polymers.

6. Photoprotective formulations according to Claim 5, **characterized in that** the additional polymers are selected from among Polyperfluoromethylisopropyl Ether, Crosspolymer Adipic Acid/ Diethylene Glycol/ Glycerin and Polysilicone-8.

7. Photoprotective formulations according to one of Claims 1 to 6, containing one or more anti-UV filters selected from the group formed of:
- cinnamic acid esters;
- dibenzoylmethane derivatives;
- 1,3,5-triazine-2,4,6-triamine derivatives;
- Diethylamino hydroxybenzoyl hexyl benzoate;
- Bis-ethyl hexyloxyphenol methoxyphenol triazine;
- Octocrylene;
- Dimethicodiethylbenzalmalonate;
- Diethylhexyl butamido triazone;
- Benzophenone derivatives;
- Phenylbenzimidazole derivatives;
- p-aminobenzoic acid derivatives;
- Methylene-bis-benzotriazolyl tetramethylbutylphenol.

8. Photoprotective formulations according to Claim 7, containing at least one anti-UV filter in micro-encapsulated form, this anti-UV filter being selected from among butyl methoxydibenzoylmethane and ethylhexyl methoxycinnamate.

9. Photoprotective formulations according to one of Claims 1 to 8, in the form of a gel, a lotion, an oil in water emulsion or a water in oil emulsion, a spray, a milk, a cream, an ointment, a foam, a stick, a vaporizer, an aerosol or any other form suitable for topical application.

10. A cosmetic formulation according to one of Claims 1 to 9, for its use in supplying effective sun screening without inducing any skin sensitization to the anti-UV filters.
